(19) Europäisches Patentamt
European Patent Office
Office européen des brevets

(11) Publication number: **0 069 494**
**B1**

(12) **EUROPEAN PATENT SPECIFICATION**

(45) Date of publication of patent specification: **14.08.85**

(51) Int. Cl.⁴: **C 07 D 317/36,**
C 07 D 317/38, B 01 J 21/00

(21) Application number: **82303186.9**

(22) Date of filing: **18.06.82**

# (54) Catalytic synthesis of cyclic carbonates.

(30) Priority: **29.06.81 US 278307**

(43) Date of publication of application:
**12.01.83 Bulletin 83/02**

(45) Publication of the grant of the patent:
**14.08.85 Bulletin 85/33**

(84) Designated Contracting States:
**CH DE FR GB IT LI**

(56) References cited:
**US-A-3 025 305**

**Angew. Chemie Int. Ed. Engl. 19 (1980), Nr. 4, p. 317-318**

**The file contains technical information submitted after the application was filed and not included in this specification**

(73) Proprietor: **THE STANDARD OIL COMPANY**
**Midland Building**
**Cleveland, Ohio 44115 (US)**

(72) Inventor: **Rinz, James Edward**
**501-B Lee Road Suite 912**
**Bedford Ohio 44146 (US)**
Inventor: **Paparizos, Christos**
**28748 Forest Road**
**Willowick Ohio 44094 (US)**
Inventor: **Herrington, Daniel Robert**
**8709 East Craig Drive**
**Chagrin Falls Ohio 44022 (US)**

(74) Representative: **Lewin, John Harvey et al**
**Elkington and Fife High Holborn House 52/54**
**High Holborn**
**London WC1V 6SH (GB)**

**EP 0 069 494 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for manufacturing cyclic carbonates. In one aspect, the invention relates to the manufacture of cyclic carbonates from oxiranes and heterocumulenes while in another aspect, the invention relates to the use in this manufacture of a novel catalytic system comprising a transition metal complex and a Lewis base.

The art is replete with various catalysts useful in the synthesis of cyclic carbonates. For example, Japanese Patent 47—31,981 teaches the synthesis of alkylene carbonates from alkylene oxides (oxiranes) and carbon dioxide in the presence of a Lewis acid (e.g. $ZnCl_2$, $AlCl_3$ and $FeCl_3$) and an organic base (e.g. pyridine, dimethylaniline and quinoline). This process is conducted at temperatures between 100 and 400°C and pressures between 19.6 and 294 bar (20 and 300 $Kg/cm^2$) with an alkylene carbonate yield of approximately 90%. Japanese Patent 51—13,720 teaches the synthesis of an alkylene carbonate from alkylene oxide in the presence of a Lewis acid (e.g. halides of zinc, iron, aluminium), a nitrogen-containing organic base (e.g. methylamine, aniline and pyridine) and water. This process is conducted at temperatures between 80 and 130°C and pressures of less than 70.9 bar(70 atms) with a product yield of approximately 90%. Japanese application 73—27,314 teaches the preparation of alkylene carbonates from alkylene oxides with carbon dioxide in the presence of a Lewis acid (e.g. halides of Zn, Al, Fe, Sn or Mg) and a base (e.g. pyridine, triethyl amine, dimethyl phenyl amine, quinoline, N-methylmorpholine and butylamine). This process is conducted at temperatures between 90 and 130°C and pressures between 13.7 and 56.9 bar (14 and 58 $Kg/cm^2$) with a product yield of approximately 90%. Angew. Chem. Int. Ed. Engl. 19 (1980) No 4, pages 317—318 teaches the synthesis of cyclic carbonates from carbon dioxide and oxiranes utilizing a catalyst composed of a mixture of a metal halide and a Lewis base.

According to this invention, cyclic carbonates are manufactured by a process comprising contacting an oxirane of the formula:

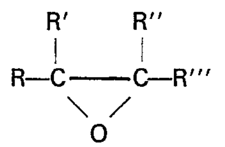

(I)

wherein R, R′, R″ and R‴ are independently H, an aliphatic group, an alicyclic group, an aromatic group or a substituted aliphatic, alicyclic or aromatic group, with a heterocumulene in the presence of a catalytic system comprising:

A. a transition metal complex of the formula:

$$L_yM \qquad \text{(II)}$$

wherein M is at least one of Ti, Zr, Hf, V, Nb and Ta;

L is an anionic ligand capable of dissociation; and

y is a number sufficient to satisfy the valency requirement of M,

but excluding titanium halide; and

B. a Lewis base.

The cyclic carbonates here used are the reaction products of the oxiranes of formula (I) and the heterocumulenes as defined below and specifically include the reaction product of an oxirane and the heterocumulene $SO_2$.

The oxiranes here used are compounds of formula (I) where R, R′, R″ and R‴ are as previously defined. The aromatic group can be monocyclic, such as phenyl, or polycyclic, either fused or nonfused. Examples of polycyclic fused aromatic groups are anthracenyl, naphthacenyl, pyrenyl and the like. Examples of polycyclic, nonfused aromatic groups are biphenyl, 2,2′-binaphthalenyl, p-terphenyl and the like. Monocyclic aromatic groups are preferred to polycyclic aromatic groups and phenyl is the preferred monocyclic aromatic group.

The alicyclic groups can be saturated or unsaturated, monocyclic or polycyclic hydrocarbons and preferably are of 3 to 12 carbon atoms. The alicyclic groups are preferred to the aromatic groups. The monocyclic, alicyclic groups include cycloalkyl and cycloalkenyl groups such as cyclopropyl, cyclobutyl, cyclobutylenyl, cyclopentenyl, cyclooctyl and the like. The polycyclic, alicyclic groups can be fused or nonfused and include bicyclo[1.1.0]butyl, bicyclo[-2.2.1]heptyl, bicyclopentyl, pentalenyl, heptalenyl and the like. Cycloalkyl groups, both mono- and polycyclic, are preferred to cycloalkyenyl groups and $C_5$ to $C_8$ monocyclic cycloalkyls are the preferred alicyclic groups.

The aliphatic groups can be linear or branched, saturated or unsaturated hydrocarbons and generally are of 1 to 12 carbon atoms. The aliphatic groups are preferred to the alicyclic groups. Representative aliphatic groups include groups. Representative aliphatic groups include alkynyl, alkenyl and alkyl groups. Representative alkynyl groups include propynyl, butynyl and pentynyl groups, while representative alkenyl groups include ethenyl, propenyl, butenyl and heptenyl groups. The preferred aliphatic groups are the alkyl

groups such as methyl, hexyl, octyl, isopropyl and t-butyl. Most preferred are the linear alkyl group having from 1 to 4 carbon atoms, i.e. methyl, ethyl, propyl and butyl.

When R′ and R″ are each aliphatic groups, they can join to form a ring and include oxirane compounds such as cyclohexene oxide.

R, R′, R″ and R‴ can independently contain one or more substituents that do not interfere with the process of the invention. Representative substituents include halogens, amides, amines, nitriles, thiols, hydroxyls and the like. The aliphatic, alicyclic and aromatic groups may also contain substituents selected from one of the other groups. For example, an aromatic or alicyclic group may contain an aliphatic substituent and the aromatic groups may contain an alicyclic substituent. Examples of this broad group of substituted groups include toluenyl, 1,3-dimethyl benzenyl, indolyl, oxacyclopenta-2,4-dienyl (furanyl), pyridinyl, oxaspiropentane and the like.

The term "oxirane" as here used means compounds containing at least one moiety of the formula:

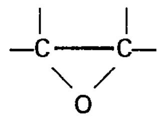

(III)

Illustrative oxiranes of this invention include oxirange (ethylene oxide), methyl oxirane (propylene oxide), phenyl oxirane (styrene oxide), dimethyl oxirane, anthracenyl oxirane, propenyl oxirane, butyl oxirane and the like. The common names for these compounds are found in the parentheses. The preferred oxiranes are oxirane (ethylene oxide) and methyl oxirane (propylene oxide).

The heterocumulenes where used are unsaturated compounds containing two or more successive double bonds such that two different atoms are joined by at least one double bond. Typically, heterocumulenes are three atom molecules such as $CO_2$, $CS_2$, COS and $SO_2$, but also include organic compounds such as $C_1$ to $C_8$ dialkyl or diaryl ketenes, $C_1$ to $C_8$ alkyl or aryl isocyanates (carbonyl amino) and $C_1$ to $C_8$ alkyl or aryl diimides. Examples of these organic heterocumulenes include diphenyl ketene, 1-(carbonyl amino) propane, 1,4-bis(carbonyl amino) benzene, p-tolylcarbodiimide and the like. Preferred heterocumulenes include $CO_2$, COS and $SO_2$ with $CO_2$ being the most preferred.

A polar solvent may be employed in the present invention, its use or non-use depending upon the nature of the reactants and the process conditions employed. The principal purpose for using the solvent is to provide a homogeneous liquid phase by solubilizing the oxirane or transition metal complex when either or both are a solid at process conditions. Other reasons for employing a solvent include monitoring process conditions, ease in handling reactants, etc. Polar solvents are those which generally produce electrically conducting solutions and cause dissociation of the solute into ions. Useful polar solvents can be selected from a wide range of compounds, such as alcohols, ketones, ethers, esters, amides, halogenated hydrocarbons and the like. A few of these solvents, such as certain alcohols and ketones, may react with the process reagents, catalysts and/or product under certain process conditions, and thus their use may be less desirable than the use of other solvents. Preferred polar solvents include tetrahydrofuran, dimethylformamide, acetonitrile, methylene chloride and chloroform.

The catalytic system of this invention consists of two parts, a transition metal complex and a Lewis base. The transition metal complex itself is composed of at least two parts, a transition metal M and a ligand L. Optionally, the complex can also contain a cycloalkadienyl or an allyl component L′ and thus be of the formula $L'_xL_yM$ wherein x is a number from 0 to about 2, y is a number from 0 to about 5 and the sum of x+y is a number sufficient to satisfy the valency requirement of M.

As noted earlier, M is a Group IVB (Ti, Zr, Hf) and/or a Group VB (V, Nb, Ta) metal and is preferably one of Ta, Zr and Hf, in particular Ta. L is an anionic ligand capable of dissociation. Typically, L is a halide ($Cl^-$, $Br^-$, $I^-$, $F^-$, preferably $Cl^-$) but can also be, among others, a hydride, A—, OA—, $NA_2$—, SA— and $SiA_3$—, where A is a $C_1$ to $C_6$ alkyl group. The optional L′ is a cycloalkadienyl group having a $C_4$ to $C_{10}$ carbon ring or an allyl group. In addition to partially satisfying the valency and charge requirements of the metal, M, L′ changes the solubility and/or stability of the metal complex and in some cases can alter the reactivity of the catalytic system through steric or electronic effects. The cycloalkadienyl ring can also contain substituents such as $C_1$ to $C_4$ alkyl or aromatic hydrocarbon groups or can be fused to one or more aromatic rings. Examples of L′ include cyclopentadienyl, pentamethylcyclopentadienyl and idenyl.

Illustrative transition metal complexes include $TaCl_5$, $TaCl_3Br_2$, $ZrCl_4$, $NbCl_5$, $HfCl_4$, $NbBr_5$, $Cp_2TiCl_2$ (wherein Cp is cyclopentadienyl), $Cp_2TiH_2$, $Cp'_2TiCl_2$ (where Cp′ is pentamethylcyclopentadienyl) and $Ta(OC_2H_5)_5$. The transition metal complexes of this invention are prepared by various methods broadly taught in the art and many such as $TaCl_5$ and $HfCl_4$ are commercially available.

While not wanting to be bound to theory, it is believed that the transition metal complex and oxirane react to form a catalytic species of the transition metal complex of the formula:

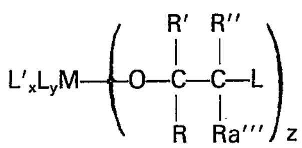

wherein the sum of x+y+z satisfies the valency requirement of M. This species is formed by the insertion of a ring-opened oxirane compound into a M-L bond of a transition metal complex precursor such as the transition metal complexes described above. Such species are typically formed *in situ* by the reaction of a transition metal complex precursor with an oxirane, but can also be prepared and isolated by the reaction of a L-substituted alcohol, such as chloropropanol, with a transition metal complex precursor such as $TaCl_5$.

The second part of this catalytic system is a Lewis base or the soluble salt of a Lewis base. Lewis bases are commonly known to those skilled in the art as compounds which are capable of donating a pair of electrons to form an adduct with a Lewis acid. Illustrative bases include pyridine, piperidine, triphenylphosphine, trialkylphosphine, sodium alkoxide, ammonium halide salts, potassium amides and lithium halides. Preferred bases are pyridine, lithium iodide and tetra-n-butylammonium bromide.

The temperature and pressure of this process can vary widely. Typically, the temperature ranges from about 20° to about 200°C, preferably from about 50° to 100°C. Subatmospheric, atmospheric or superatmospheric pressure can be used but typically, the pressure ranges from about 1 to about 101 bar (about 1 to about 100 atms), preferably from about 20 to about 41 bar (about 20 to about 40 atms). The mixture is preferably agitated while being heated.

The concentration of the transition metal complex typically ranges from about 0.001 to 100 parts per 1,000 parts of oxiranea and preferably ranges from about 0.1 to 10 parts of transition metal complex per 1,000 parts of oxirane. The concentration of the Lewis base is typically from 0.001 to 100 parts per 1,000 parts of oxirane and preferably ranges from about 0.2 to about 50 parts of base per 1,000 parts of oxirane.

The process can be conducted in a homogeneous or heterogeneous mode. A homogeneous mode, i.e. the reactants and catalyst in one phase, can be accomplished by solubilizing those reactants which remain in a solid phase at process conditions in a polar solvent. A heterogeneous mode can be accomplished by incorporating the components of the catalytic system onto an appropriate support.

The invention is illustrated by the following Examples.

All of the experiments were conducted in a 300 ml Parr Hastelloy C autoclave equipped with a stirring mechanism and a temperature control. The specific metal complexes, bases and reaction times are reported in Table I. All experiments were conducted in 50 ml of tetrahydrofuran except example 8, in which 25 ml were used. The procedure of Example 1 was used throughout unless otherwise indicated.

Example 1

Tetrahydrofuran (50 ml) was placed in the autoclave and 5.0 mmol of the tantalum pentachloride was dissolved in the solvent. Next, 0.20 moles of methyl oxirane (propylene oxide) and 12.5 mmol of pyridine were added sequentially. The autoclave was sealed and briefly purged with carbon dioxide. While continuously stirring the mixture, the autoclave was heated to a temperature of 60°C and subsequently charged with carbon dioxide to a total pressure of 34.5 bar gauge (500 psig). The reaction was continued for 5 hours at which time a sample was drawn and analyzed by IR spectrophotometry and NMR. The results are reported in Table I.

Examples A to E and Examples 2 to 10

Examples A to E are controls which illustrate the significance of using specific combinations of a transition metal complex and a Lewis base. When either the metal complex or the base is used alone in the process, the yield of product is negligible.

Examples 2 to 10 illustrate the use of various transition metal complexes in combination with various Lewis bases. The percent yields were determined by NMR (integration) and no by-products were observed. These Examples illustrate that the present invention is extremely selective, employs very mild conditions and has high yields. Comparing the percent product manufactured in the controls to those of Examples 1 to 10 illustrates the superior results of the latter over the former.

The transition metal complex in Example 9 includes the cyclopentadienyl group. The result was an extremely high yield of product (greater than 99%). Examples 8 and 10 illustrate that these catalytic systems are not limited to methyl oxirane (propylene oxide) since butylenyl oxirange (butadiene monooxide) and butylenyl dioxirane (butadiene diepoxide) are found to be excellent starting oxiranes as their use results in yields greater than 99% product.

TABLE I

| Example | Metal complex | mmol of catalyst | Base | mmol of Base | Reaction time-hrs | Percent yield |
|---|---|---|---|---|---|---|
| A | $TaCl_5$ | 5.0 | — | — | 5.0 | Trace |
| B | — | — | Pyridine | 25.0 | 5.0 | 0 |
| C | — | — | Tetra-n-butyl-ammonium bromide | 12.5 | 1.0 | 0 |
| D | — | — | Lithium Iodide | 5.0 | 1.0 | 0 |
| E[1] | $Cp_2TiCl_2$ | 5.0 | — | — | 3.0 | 4 |
| 1 | $TaCl_5$ | 5.0 | Pyridine | 25.0 | 5.0 | >99 |
| 2 | $TaCl_5$ | 5.0 | Triphenyl Phosphine | 25.0 | 5.0 | 52 |
| 3 | $TaCl_5$ | 5.0 | Tetra-n-butyl-ammonium bromide | 12.5 | 5.0 | >99 |
| 4 | $ZrCl_4$ | 5.0 | " | 12.5 | 1.0 | 95 |
| 5 | $NbCl_5$ | 5.0 | " | 12.5 | 1.0 | 20 |
| 6 | $HfCl_4$ | 5.0 | " | 12.5 | 1.0 | >99 |
| 7 | $TaCl_5$ | 5.0 | Lithium Iodide | 12.5 | 1.0 | >99 |
| 8[2] | $TaCl_5$ | 2.5 | Tetra-n-butyl-ammonium bromide | 6.2 | 22.0 | >99 |
| 9[3] | $Cp_2TiCl_2$ | 5.0 | " | 12.5 | 2.0 | ≈99 |
| 10[4] | $TaCl_5$ | 2.5 | " | 12.5 | 4.0 | >99 |

[1] The reaction temperature was 150°C, the reaction pressure was 17.2 bar gauge (250 psig) and Cp=cyclopentadienyl ($C_5H_5$).
[2] Butadiene monoxide (380 mmol) was the oxirane used in this reaction. The reaction temperature was 80°C and the reaction pressure was 34.5 bar gauge (500 psig).
[3] Cp=Cyclopentadienyl.
[4] Butadiene diepoxide (290 mmol) was the oxirane used in this reaction. The reaction temperature was 100°C.

Table II is a comparison of the reaction rates of an $AlCl_3$ system and a $TaCl_5$ system (invention). For both systems the following process conditions were employed: 2,000 mmol of methyl oxirane, 5.0 mmol of complex and 12.5 moles of tetra-n-butylammonium bromide were contacted in 50.0 mls of tetrahydrofuran at 60°C and 34.5 bar gauge (500 psig). The present invention clearly demonstrates a more expedient and efficient yield of product than that of the $AlCl_3$ system under indentical process conditions.

TABLE II

| Example | Catalyst | Reaction time (min.) | Percent product |
|---|---|---|---|
| A | $AlCl_3$ | <1 | 11 |
| B | " | 10 | 16 |
| C | " | 20 | 23 |
| D | " | 30 | 24 |
| E | " | 60 | 31 |
| 1 | $TaCl_5$ | <1 | 8 |
| 2 | " | 10 | 31 |
| 3 | " | 20 | 49 |
| 4 | " | 30 | 62 |
| 5 | " | 60 | 91 |

**Claims**

1. A process for manufacturing cyclic carbonates, comprising contacting an oxirane of the formula:

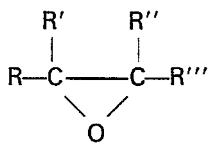

(I)

wherein R, R′, R″ and R‴ are independently H, an aliphatic group, an alicyclic group, an aromatic group or a substituted aliphatic, alicyclic or aromatic group, with a heterocumulene in the presence of a catalytic system comprising:

A. a transition metal complex of the formula:

$$L_yM \quad \text{(II)}$$

wherein M is at least one of Ti, Zr, Hf, V, Nb and Ta;

L is an anionic ligand capable of dissociation; and

y is a number sufficient to satisfy the valency requirement of M,

but excluding titanium halide; and

B. a Lewis base.

2. A process as claimed in claim 1, wherein the transition metal complex of formula (II) includes L′ and is thus of the formula:

$$L'_xL_yM$$

wherein L, M, y are as defined in claim 1;

L′ is selected from a cycloalkadienyl group having a $C_4$ to $C_{10}$ carbon ring and an allyl group;

x is a number from 0 to 2; and

the sum of x+y is a number sufficient to satisfy the valency requirements of M.

3. A process for manufacturing cyclic carbonates, comprising contacting an oxirane of the formula (I), as defined in claim 1, with a heterocumulene in the presence of a catalytic system comprising:

A. a species of a transition metal complex of the formula:

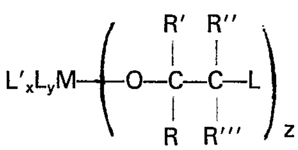

wherein M and L are as defined in claim 1;

L′ is selected from a cycloalkadienyl group having a $C_4$ to $C_{10}$ carbon ring and an allyl group;
x is a number from 0 to 2;
y is a number from 0 to 5;
z is a number from 0 to 5; and
the sum of x+y+z is a number sufficient to satisfy the valence requirements of M; and
B. a Lewis base.

4. A process as claimed in any of claims 1 to 3, wherein M is selected from Ta, Zr and Hf.

5. A process as claimed in claim 4, wherein M is Ta.

6. A process as claimed in any of claims 1 to 5, wherein L is selected from $Cl^-$, $Br^-$, $I^-$ and $F^-$.

7. A process as claimed in claim 6, wherein L is $Cl^-$.

8. A process as claimed in any of claims 2 to 7, wherein L′ is a cyclopentadienyl group.

9. A process as claimed in any of claims 1 to 8, wherein R, R′, R″ and R‴ are independently a $C_1$ to $C_{12}$ aliphatic or $C_3$ to $C_{12}$ alicyclic group.

10. A process as claimed in claim 9, wherein R, R′, R″ and R‴ are independently a $C_1$ to $C_4$ aliphatic group.

11. A process as claimed in any of claims 1 to 10, wherein the oxirane is selected from ethylene oxide and propylene oxide.

12. A process as claimed in any of claims 1 to 11, wherein the base is selected from pyridine, tetra-n-butylammonium bromide and lithium iodide.

13. A process as claimed in claim 12, wherein the base is a bromide ion.

14. A process as claimed in any of claims 1 to 13, wherein the concentration of the transition metal complex is from 0.001 to 100 parts per 1,000 parts of oxirane and the concentration of the Lewis base is from 0.001 to 100 parts per 1,000 parts of oxirane.

15. A process as claimed in claim 14, wherein the concentration of the transition metal complex is from 0.1 to 10 parts per 1,000 parts of oxirane and the concentration of the Lewis base is from 0.2 to 50 parts per 1,000 parts of oxirane.

16. A process as claimed in any of claims 1 to 15, wherein the temperature is from 50 to 100°C.

17. A process as claimed in any of claims 1 to 16, wherein the pressure is from 20 to 41 bar (20 to 40 atms).

18. A catalytic system for manufacturing cyclic carbonates from oxiranes and heterocumulenes, the system comprising:
A. a transition metal complex of the formula:

$$L'_xL_yM$$

wherein M is at least one of Ti, Zr, Hf, V, and Nb, and Ta;
L is an anionic ligand capable of dissociation;
L′ is selected from a cycloalkadienyl group having a $C_4$ to $C_{10}$ carbon ring and an allyl group;
x is a number from 0 to 2;
y is a number from 0 to 5; and
the sum of x+y is a number sufficient to satisfy the valency requirement of M,
but excluding titanium halide; and
B. a Lewis base.

19. A catalytic system for manufacturing cyclic carbonates from oxiranes and heterocumulenes, the system comprising:
A. a species of a transition metal complex of the formula:

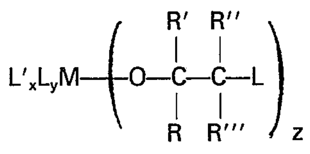

wherein M is at least one of Ti, Zr, Hf, V, Nb and Ta;
L is an anionic ligand capable of dissociation;
L′ is selected from a cycloalkadienyl group having a $C_4$ to $C_{10}$ carbon ring or an allyl group;
x is a number from 0 to 2;
y is a number from 0 to 5;
z is a number from 0 to 5; and
the sum of x+y+z is a number sufficient to satisfy the valence requirements of M; and
B. a Lewis base.

**Patentansprüche**

1. Verfahren zur Herstellung cyclischer Carbonate, dadurch gekennzeichnet, daß ein Oxiran der Formel

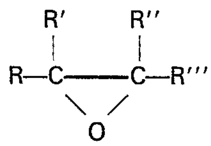 (I)

worin R, R', R'' und R''' unabhängig voneinander Wasserstoff, eine aliphatische Gruppe, eine alicyclische Gruppe, eine aromatische Gruppe oder eine substituierte aliphatische, alicyclische oder aromatische Gruppe sind, mit einem Heterocumulen in Anwesenheit eines katalytischen Systems in Berührung gebracht wird, das enthält:

A. ein Übergangsmetallkomplex der Formel

$$L_yM \qquad \text{(II)}$$

worin M mindestens eines der Elemente Ti, Zr, Hf, V, Nb und Ta;

L ein anionischer, zur Dissoziation befähigter Ligand; und

y eine Zahl ist, die genügt, um die Valenzen von M abzusättigen, ausgenommen jedoch Titaniumhalogenid; und

B. eine Lewis-Base.

2. Verfahren gemäß Anspruch 1, dadurch gekennzeichnet, daß der Übergangsmetallkomplex der Formel (II) L' umfaßt und demnach der Formel

$$L'_xL_yM$$

entspricht, worin L, M, und y die im Anspruch 1 angegebene Bedeutung haben;

L' aus einer Cycloalkadienyl-Gruppe mit einem Kohlenstoffring mit 4 bis 10 Kohlenstoffatomen und eine Allylgruppe ausgewählt;

x eine Zahl von 0 bis 2; und

die Summe x+y eine Zahl ist, die genügt, um die Wertigkeitsanroderungen von M zu befriedigen.

3. Verfahren zur Herstellung cyclischer Carbonate, dadurch gekennzeichnet, daß ein Oxiran der Formel (I), wie im Anspruch 1 definiert, mit einem Heterocumulen in Anwesenheit eines katalytischen Systems in Berührung gebracht wird, das enthält:

A. eine Art eines Übergangsmetallkomplexes der Formel

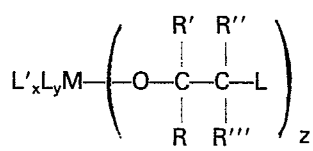

worin M und L die gleiche Bedeutung wie im Anspruch 1 haben;

L' ausgewählt ist aus einer Cycloalkadienyl-Gruppe mit einem Ring mit 4 bis 10 Kohlenstoffatomen und einer Allylgruppe;

x eine Zahl von 0 bis 2;

y eine Zahl von 0 bis 5;

z eine Zahl von 0 bis 5 und

die Summe x+y+z eine Zahl ist, die genügt, um den Wertigkeitsanforderungen von M zu genügen; und

B. eine Lewis-Base.

4. Verfahren gemäß einem der Ansprüche 1 bis 3, dadurch gekennzeichnet, daß M ausgewählt ist aus Ta, Zr und Hf.

5. Verfahren gemäß Anspruch 4, dadurch gekennzeichnet, daß M Ta ist.

6. Verfahren gemäß einem der Ansprüche 1 bis 5, dadurch gekennzeichnet, daß L ausgewählt ist aus $Cl^-$, $Br^-$, $I^-$ und $F^-$.

7. Verfahren gemäß Anspruch 6, dadurch gekennzeichnet, daß L $Cl^-$ ist.

8. Verfahren gemäß einem der Ansprüche 2 bis 7, dadurch gekennzeichnet, daß L' eine Cyclopentadienyl-Gruppe ist.

9. Verfahren gemäß einem der Ansprüche 1 bis 8, dadurch gekennzeichnet, daß R, R', R'' und R''' unabhängig voneinander eine $C_1$ bis $C_{12}$-aliphatische oder $C_3$ bis $C_{12}$-alicyclische Gruppe ist.

10. Verfahren gemäß Anspruch 9, dadurch gekennzeichnet, daß R, R', R'' und R''' unabhängig voneinander eine $C_1$ bis $C_4$ aliphatische Gruppe ist.

11. Verfahren gemäß einem der Ansprüche 1 bis 10, dadurch gekennzeichnet, daß das Oxiran ausgewählt ist aus Ethylenoxid und Propylenoxid.

12. Verfahren gemäß einem der Ansprüche 1 bis 11, dadurch gekennzeichnet, daß die Base ausgewählt ist aus Pyridin, Tetra-n-butyl-ammoniumbromid und Lithiumjodid.

13. Verfahren gemäß Anspruch 12, dadurch gekennzeichnet, daß die Base ein Bromidion ist.

14. Verfahren gemäß einem der Ansprüche 1 bis 13, dadurch gekennzeichnet, daß die Konzentration des Übergangsmetallkomplexes im Bereich von 0.001 bis 100 Teile pro 1000 Teile des Oxirans und die Konzentration der Lewis-Base im Bereich von 0.001 bis 100 Teile pro 1000 Teile des Oxirans beträgt.

15. Verfahren gemäß Anspruch 14, dadurch gekennzeichnet, daß die Konzentration des Übergangsmetallkomplexes im Bereich von 0.1 bis 10 Teilen pro 1000 Teile des Oxirans und die Konzentration der Lewis-Base im Bereich von 0.2 bis 50 Teilen pro 1000 Teile des Oxirans beträgt.

16. Verfahren gemäß einem der Ansprüche 1 bis 15, dadurch gekennzeichnet, daß die Temperatur im Bereich von 50 bis 100°C liegt.

17. Verfahren gemäß einem der Ansprüche 1 bis 16, dadurch gekennzeichnet, daß der Druck im Bereich von 20 bis 40 Bar (20 bis 40 Atmosphären) liegt.

18. Ein katalytisches System zur Herstellung cyclischer Carbonate aus Oxiranen und Heterocumulenen, dadurch gekennzeichnet, daß das System enthält:

A. einen Übergangsmetallkomplex der Formel

$$L'_xL_yM$$

worin M mindestens eines von Ti, Zr, Hf, V, Nb und Ta ist;

L ein anionischer, zur Dissoziation gefähigter Ligand;

L' ausgewählt ist aus einer Cycloalkadienyl-Gruppe mit 4 bis 10 Ringkohlenstoffatomen und einer Allylgruppe;

x eine Zahl von 0 bis 2;

y eine Zahl von 0 bis 5; und

die Summe x+y eine Zahl ist, die genügt, um den Wertigkeitsanforderungen von M zu genügen, jedoch unter Ausschluß von Titanhalogenid; und

B. eine Lewis-Base.

19. Ein katalytisches System zur Herstellung cyclischer Carbonate aus Oxiranen und Heterocumulenen, dadurch gekennzeichnet, daß das System enthält:

A. eine Art eines Übergangsmetallkomplexes der Formel

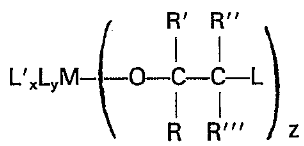

worin M mindestens eines von Ti, Zr, Hf, V, Nb und Ta;

L ein anionischer, zur Dissoziation befähigter Ligand;

L' aus einer Cycloalkadienyl-Gruppe mit 4 bis 10 Ringkohlenstoffatomen oder einer Allylgruppe ausgewählt ist;

x eine Zahl von 0 bis 2;

y eine Zahl von 0 bis 5;

z eine Zahl von 0 bis 5; und

die Summe x+y+z eine Zahl ist, die genügt, um den Wertigkeitsanforderungen von M zu genügen; und

B. eine Lewis-Base.

**Revendications**

1. Procédé de fabrication de carbonates cycliques consistant à mettre en contact un oxirane répondant à la formule:

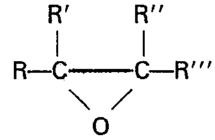

(I)

dans laquelle R, R', R'' et R''' sont indépendamment H, un groupe aliphatique, un groupe alicyclique, un groupe aromatique ou un groupe aliphatique, alicyclique ou aromatique substitué, avec un hétérocumulène en présence d'un système catalytique comprenant:

A. un complexe de métal de transition répondant à la formule:

$$L_yM \quad \text{(II)}$$

dans laquelle M est au moins l'un parmi Ti, Zr, Hf, V, Nb et Ta;

L est un ligand anionique capable de dissociation; et

y est un nombre suffisant pour satisfaire aux exigences de valence de M,

mais à l'exclusion d'un halogénure de titane; et

B. une base de Lewis.

2. Procédé selon la revendication 1, dans lequel le contexte de métal de transition répondant à la formule (II) comprend L', et répond ainsi à la formule:

$$L'_xL_yM$$

dans laquelle L, M, y sont tels que définis à la revendication 1;

L' est choisi parmi un groupe cycloalkadiényle ayant un cycle carboné en $C_4$ à $C_{10}$ et un groupe allyle;

x est un nombre de 0 à 2; et

la somme x+y est un nombre suffisant pour satisfaire aux exigences de valence de M.

3. Procédé de fabrication de carbonates cycliques consistant à mettre en contact un oxirane répondant à la formule (I) tel que défini dans la revendication 1, avec un hétérocumulène en présence d'un système catalytique comprenant:

A. une espèce d'un complexe de métal de transition répondant à la formule

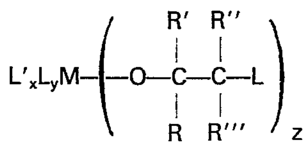

dans laquelle M et L sont tels que définis dans la revendication 1;

L' est choisi parmi un groupe cycloalkadiényle ayant un cycle carboné en $C_4$ à $C_{10}$ et un groupe allyle;

x est un nombre de 0 à 2;

y est un nombre de 0 à 5;

z est un nombre de 0 à 5; et

la somme x+y+z est un nombre suffisant pour satisfaire aux exigences de valence de M; et

B. une base de Lewis.

4. Procédé selon l'une des revendications 1 à 3, dans lequel M est choisi parmi Ta, Zr et Hf.

5. Procédé selon la revendication 4, dans lequel M est Ta.

6. Procédé selon l'une des revendications 1 à 5, dans lequel L est choisi parmi $Cl^-$, $Br^-$, $I^-$ et $F^-$.

7. Procédé selon la revendication 6, dans lequel L est $Cl^-$.

8. Procédé selon l'une des revendications 2 à 7, dans lequel L' est un groupe cyclopentadiényle.

9. Procédé selon l'une des revendications 1 à 8, dans lequel R, R', R'' et R''' sont indépendamment un groupe aliphatique en $C_1$ à $C_{12}$ ou alicyclique en $C_3$ à $C_{12}$.

10. Procédé selon la revendication 9, dans lequel R, R', R'' et R''' sont indépendamment un groupe aliphatique en $C_1$ à $C_4$.

11. Procédé selon l'une des revendications 1 à 10, dans lequel l'oxirane est choisi parmi l'oxyde d'éthylène et l'oxyde de propylène.

12. Procédé selon l'une des revendications 1 à 11, dans lequel la base est choisie parmi la pyridine, le bromure de tétra-n-butylammonium et l'iodure de lithium.

13. Procédé selon la revendication 12, dans lequel la base est un ion bromure.

14. Procédé selon l'une des revendications 1 à 13, dans lequel la concentration du complexe de métal de transition est de 0,001 à 100 parties pour 1000 parties d'oxirane et la concentration de la base de Lewis est de 0,001 à 100 parties pour 1000 parties d'oxirane.

15. Procédé selon la revendication 14, dans lequel la concentration du complexe de métal de transition est de 0,1 à 10 parties pour 1000 parties d'oxirane et la concentration de la base de Lewis est de 0,2 à 50 parties pour 1000 parties d'oxirane.

16. Procédé selon l'une des revendications 1 à 15, dans lequel la température est de 50 à 100°C.

17. Procédé selon l'une des revendications 1 à 16, dans lequel la pression est de 20 à 41 bars (20 à 40 atms).

18. Système catalytique pour la fabrication de carbonates cycliques à partir d'oxiranes et d'hétérocumulènes, le système comprenant:

A. un complexe de métal de transition répondant à la formule:

$$L'_xL_yM$$

dans laquelle M est au moins l'un parmi Ti, Zr, Hf, V, Nb et Ta;

L est un ligand anionique capable de dissociation;

L' est choisi parmi un groupe cycloalkadiényle ayant un cycle carboné en $C_4$ à $C_{10}$ et un groupe allyle;

x est un nombre de 0 à 2;

y est un nombre de 0 à 5; et

la somme x+y est un nombre suffisant pour satisfaire aux exigences de valence de M, mais à l'exclusion d'un halogénure de titane; et

B. une base de Lewis.

19. Système catalytique pour la fabrication de carbonates cycliques à partir d'oxiranes et d'hétérocumulènes, le système comprenant:

A. un espèce d'un complexe de métal de transition répondant à la formule:

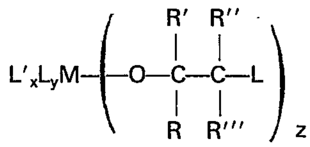

dans laquelle M est au moins l'un parmi—Ti, Zr, Hf, V, Nb et Ta;

L est un ligand anionique capable—de dissociation;

L' est choisi parmi un groupe cycloalkadiényle ayant un cycle carboné en $C_4$ à $C_{10}$ et un groupe allyle;

x est un nombre de 0 à 2;

y est un nombre de 0 à 5;

z est un nombre de 0 à 5; et

la somme x+y+z est un nombre suffisant pour satisfaire aux exigences de valence de M; et

B. une base de Lewis.